Europäisches Patentamt

(19) European Patent Office

Office européen des brevets

(11) Publication number : **0 331 415 B1**

# EUROPEAN PATENT SPECIFICATION

(45) Date of publication of patent specification :
03.06.92 Bulletin 92/23

(51) Int. Cl.⁵ : **A61K 7/16**

(21) Application number : **89301954.7**

(22) Date of filing : **28.02.89**

(54) **Anticalculus oral compostions.**

The file contains technical information
submitted after the application was filed and
not included in this specification

(30) Priority : **01.03.88 GB 8804817**

(43) Date of publication of application :
**06.09.89 Bulletin 89/36**

(45) Publication of the grant of the patent :
**03.06.92 Bulletin 92/23**

(84) Designated Contracting States :
**AT BE CH DE ES FR GB GR IT LI NL SE**

(56) References cited :
**EP-A- 0 236 290**
**EP-A- 0 249 398**
**DE-A- 3 629 504**
**GB-A- 2 201 593**

(72) Inventor : **Carter, Peter**
**Briarfield, The Rake**
**Burton Wirral L64 5TL (GB)**
Inventor : **Cummins, Diane**
**55 Caldy Road**
**West Kirby Wirral L48 2HF (GB)**
Inventor : **Jones, David Alan Kenneth**
**Mendips, 2 Spital Road**
**Bebington Wirral L63 9JE (GB)**
Inventor : **Lowry, Michael Richard**
**46 St Peters Way**
**Mickle Trafford Chester CH2 4EJ (GB)**

(74) Representative : **van Gent, Jan Paulus et al**
**Unilever N.V., Patent Division Postbus 137**
**NL-3130 AC Vlaardingen (NL)**

(73) Proprietor : **UNILEVER PLC**
**Unilever House Blackfriars P.O. Box 68**
**London EC4P 4BQ (GB)**
(84) **GB**

Proprietor : **UNILEVER NV**
**Burgemeester s'Jacobplein 1 P.O. Box 760**
**NL-3000 DK Rotterdam (NL)**
(84) **BE CH DE ES FR GR IT LI NL SE AT**

## Description

This invention relates to anticalculus oral compositions, more particularly anticalculus toothpastes containing a water-soluble pyrophosphate salt.

It is well known that the soluble pyrophosphate salts have anticalculus activity. Patents and patent applications that have been published relating to anticalculus toothpastes comprising a soluble pyrophosphate salt include US-A-4 684 518, US-A-4 627 977, US-A-4 590 066, US-A-4 515 772, GB-A-2 182 244, EP-A-295 116, EP-A-251 591, EP-A-249 398, EP-A-236 827 and EP-A-236 290. The disclosures of these publications are included herein by reference.

The present invention relates to an anticalculus toothpaste composition based on the use of a water-soluble pyrophosphate and which is particularly efficacious.

The anticalculus toothpaste of the invention comprises an abrasive agent and an amount of pyrophosphate salt sufficient to provide at least 2.5% $P_2O_7^{4-}$, and is characterised by having a pH, measured directly on the paste, of at least 7.6, preferably at least 8.0, which rises by at least 0.45, preferably at least 0.60, on dilution of the toothpaste with distilled carbon dioxide-free water in the proportion of 10g of toothpaste to 30ml of water. The pH value on dilution is determined in the following way. Toothpaste (10g) is added to a tall form 150ml glass beaker. Distilled carbon dioxide-free water (10ml) is added and by stirring for two minutes using a laboratory stirrer (such as a Heidolf mixer at 1500ppm) the toothpaste is thoroughly dispersed in the water. After one minute a further 10ml of the water is added and the mixture stirred again for two minutes. After 1 minute a final 10ml of the water is added and the mixture stirred for another two minutes. The pH of the toothpaste slurry is measured after one minute. All pH measurements are made at 20°C.

This improved composition is based on our discovery that the effectiveness of a toothpaste in inhibiting the formation of dental calculus is enhanced by increased pH.

The abrasive agent of the toothpaste is preferably a silica abrasive but may be any of those referred to in any of the publications referred to above. Silica abrasives are particularly compatible with soluble pyrophosphate anticalculus agents. Suitable silica abrasives are well known to those skilled in the art. These include silica xerogels such as Syloid 63 available from Grace, precipitated silicas such as Zeodent 119 from Huber, and bifunctional silicas such as Sident 15 from Degussa. Examples of other abrasives disclosed in the prior published patent specifications referred to above include beta phase calcium pyrophosphate, alumina, insoluble metaphosphate and thermosetting resins. The amount of abrasive will generally be from about 5% to about 50%, especially from 8% to 25%, bv weight of the toothpaste.

We have found that some ingredients disclosed in prior art anticalculus compositions containing soluble pyrophosphates tend to give a toothpaste a pH of less than 7.6 and/or limit an increase in pH on dilution of the toothpaste to less than 0.45. Such ingredients include the humectant glycerol, polycarboxylic materials such as the Gantrez resins, and acid buffering components such as mono- and di-sodium phosphates. Therefore sorbitol is the preferred humectant and acidic polycarboxylic materials and acidic buffers are preferably absent.

The pyrophosphate employed in the toothpaste of the invention preferably consists of tetrapotassium pyrophosphate or tetrasodium pyrophosphate or a mixture of tetrapotassium pyrophosphate and tetrasodium pyrophosphate. When employing a mixture of tetrapotassium pyrophosphate and tetrasodium pyrophosphate the weight of the tetrapotassium pyrophosphate preferably ranges from about 0.7% to about 4% by weight and the tetrasodium pyrophosphate from about 0.6% to about 7% by weight. The choice of such preferred amounts, while meeting the above condition regarding the minimum amount of pyrophosphate, is particularly advantageous in providing a level of pyrophosphate which gives a high degree of efficacy and yet avoids the use of levels of tetrapotassium pyrophosphate which may give an unsatisfactory salty taste, or levels of tetrasodium pyrophosphate which, due to its lower solubility, can give rise to an unacceptable grittiness. The preferred ratio of tetrapotassium pyrophosphate to tetrasodium pyrophosphate is from about 1.2:1 to 3.7:1 by weight. The desired pH of the composition is preferably achieved through an appropriate choice of the pyrophosphate material or by the addition of an alkaline agent. The acid pyrophosphates are not employed since they tend to give products having a lower pH.

The toothpaste of the invention also preferably comprises a fluoride-ion source sufficient to supply from 50ppm to 3500ppm, preferably 500ppm to 2000ppm, of fluoride ions. It is well known that fluoride ions have an anticaries effect and can also inhibit hydrolysis of pyrophosphate by the pyrophosphatase enzyme. The fluoride-ion source is preferably sodium fluoride or sodium monofluorophosphate.

The toothpaste of the invention will contain other common ingredients well known to those in the art, including an humectant, a binder or thickening agent, surfactant and flavouring agent.

The preferred humectant of toothpastes according to the present invention is sorbitol, which is usually incorporated in the form of an approximately 70% by weight aqueous solution. However, other humectants may be used provided they do not substantially inhibit pH rise on dilution of the toothpaste with distilled carbon dioxide-free water as described above. The amount of the humectant will generally range from about 10% to about 85% by weight of the toothpaste. Preferred toothpastes of the invention are glycerol-free.

Numerous binding or thickening agents have been indicated for use in toothpastes, preferred ones being sodium carboxymethylcellulose and xanthan gum. Others include natural gum binders such as gum tragacanth, gum karaya and gum arabic, Irish moss, alginates and carrageenans. Various forms of silica are also used for thickening toothpastes, e.g. fumed silicas, silica aerogels and precipitated silicas. Mixtures of binders or thickeners may be used. The amount of binder or thickener included in a toothpaste is generally between 0.1 and 10% by weight.

It is usual to include a surfactant in a toothpaste and again the literature discloses a wide variety of suitable materials. Surfactants which have found wide use in practice are sodium lauryl sulphate, sodium dodecylbenzene sulphonate and sodium laurylsarcosinate. Other anionic surfactants may be used as well as other types such as cationic, amphoteric and non-ionic surfactants. Surfactants are usually present in an amount of from 0.5 to 5% by weight of the toothpaste.

Flavours that are usually used in toothpastes are those based on oils of spearmint and peppermint. Examples of other flavouring materials used are menthol, clove, wintergreen, eucalyptus and aniseed. An amount of from 0.1% to 5% by weight of the toothpaste is a suitable amount of flavour to incorporate in a toothpaste.

The toothpaste of the invention may include a wide variety of optional ingredients. These include an antiplaque agent such as an antimicrobial compound for example chlorhexidine or 2,4,4′ -trichloro-2 ′-hydroxydiphenyl ether, or a copper salt (see EP-A-0 038 867); another anti-tartar ingredient such as zinc citrate or other zinc salt (see US-A-4 100 269); further thickening or structuring agents such as polyethylene glycol 1500; a sweetening agent such as saccharin; an opacifying agent, such as titanum dioxide; a preservative, such as formalin; or a colouring agent.

The invention also relates to a method of preparing a toothpaste composition according to the invention which comprises mixing together the components in a conventional manner.

For a fuller discussion of the formulation of toothpastes reference is made to Harry's Cosmeticology, Seventh Edition, 1982, Edited by J B Wilkinson and R J Moore, pages 609 to 617.

The following Examples illustrate the invention. Percentages are by weight.

<u>Example 1</u>

An anticalculus toothpaste of the following composition was prepared.

| Ingredient | % |
|---|---|
| Abrasive silica[1] | 10.00 |
| Thickening silica[2] | 8.00 |
| Sorbitol syrup (70% solution) | 40.00 |
| Polyethylene glycol 1500 | 5.00 |
| Sodium lauryl sulphate | 1.70 |
| Sodium dodecylbenzene sulphonate | 0.50 |
| Xanthan gum | 0.60 |
| Tetrapotassium pyrophosphate | 3.10 |
| Tetrasodium pyrophosphate | 2.50 |
| Sodium fluoride | 0.24 |
| Sodium saccharin | 0.20 |
| Titanium dioxide | 1.00 |
| Flavour | 1.20 |
| Formalin | 0.04 |
| Deionised water | to 100.00 |

1      - a silica gel sold by Joseph Crosfield & Sons Ltd as Gasil 200

2      - a silica gel sold by Joseph Crosfield & Sons Ltd as Gasil 23

The two pyrophosphate salts were present in a total amount corresponding to 3.3% $P_2O_7^{4-}$.

The toothpaste had a pH of 8.08. On dilution of 10g of the toothpaste with 30ml of distilled carbon dioxide-free water in the manner described above the pH rose to 8.72.

The toothpaste retained a high level of available fluoride and pyrophosphate when stored for 3 months at 0°C, 20°C and 37°C.

Example 2

An anticalculus toothpaste of the following composition was prepared.

4

| Ingredient | % |
|---|---|
| Abrasive silica[1] | 10.00 |
| Thickening silica[2] | 8.50 |
| Sorbitol syrup (70% solution) | 40.50 |
| Polyethylene glycol 1500 | 5.00 |
| Sodium lauryl sulphate | 1.50 |
| Xanthan gum | 0.60 |
| Tetrapotassium pyrophosphate | 3.10 |
| Tetrasodium pyrophosphate | 2.50 |
| Sodium fluoride | 0.24 |
| Sodium saccharin | 0.20 |
| Titanium dioxide | 1.00 |
| Flavour | 1.00 |
| Deionised water | 25.86 |

1   - as Example 1

2   - a precipitated silica sold by Degussa as Sident 22S

The two pyrophosphate salts were present in a total amount corresponding to 3.3% $P_2O_7^{4-}$.

The toothpaste had a pH of 8.04. On dilution of 10g of the toothpaste with 30ml of distilled carbon dioxide-free water in the manner described above the pH rose to 8.54.

The toothpaste retained a high level of available fluoride and pyrophosphate when stored for 3 months at 0°C, 20°C and 37°C.

Example 3

An anticalculus toothpaste of the following composition was prepared.

| Ingredient | % |
|---|---|
| Abrasive silica[1] | 10.00 |
| Thickening silica[2] | 8.00 |
| Sorbitol syrup (70% solution) | 40.00 |
| Polyethylene glycol 1500 | 5.00 |
| Sodium lauryl sulphate | 1.70 |
| Sodium dodecylbenzene sulphonate | 0.50 |
| Xanthan gum | 0.60 |
| Tetrapotassium pyrophosphate | 3.10 |
| Tetrasodium pyrophosphate | 2.50 |
| Zinc citrate trihydrate | 0.50 |
| Sodium fluoride | 0.24 |
| Sodium saccharin | 0.20 |
| Titanium dioxide | 1.00 |
| Flavour | 1.20 |
| Formalin | 0.04 |
| Deionised water | to 100.00 |

1   – as Example 1

2   – as Example 1

The two pyrophosphate salts were present in a total amount corresponding to 3.3% $P_2O_7^{4-}$.

The toothpaste had a pH of 8.10. On dilution of 10g of the toothpaste with 30ml of distilled carbon dioxide-free water in the manner described above the pH rose to 8.73.

The toothpaste retained a high level of avalaible fluoride, pyrophosphate and zinc ions when stored for 3 months at 0°C, 20°C and 37°C.

Example 4

An anticalculus toothpaste of the following composition was prepared.

| Ingredient | % |
| --- | --- |
| Abrasive silica[1] | 10.00 |
| Thickening silica[2] | 8.50 |
| Sorbitol syrup (70% solution) | 40.00 |
| Polyethylene glycol 1500 | 5.00 |
| Sodium lauryl sulphate | 1.50 |
| Xanthan gum | 0.60 |
| Tetrapotassium pyrophosphate | 3.10 |
| Tetrasodium pyrophosphate | 2.50 |
| Zinc citrate trihydrate | 0.50 |
| Sodium fluoride | 0.24 |
| Sodium saccharin | 0.20 |
| Titanium dioxide | 1.00 |
| Flavour | 1.00 |
| Deionised water | 25.86 |

[1] - as Example 1

[2] - as Example 2

The two pyrophosphate salts were present in a total amount corresponding to 3.3% $P_2O_7^{4-}$.

The toothpaste had a pH of 7.86. On dilution of 10g of the toothpaste with 30ml of distilled carbon dioxide free water in the manner described above the pH rose to 8.41.

The toothpaste retained a high level of available fluoride, pyrophosphate and zinc ions when stored for 3 months at 0°C, 20°C and 37°C.

## Claims

1. A toothpaste for inhibiting the formation of dental calculus comprising an abrasive agent and an amount of tetrapyrophosphate salt sufficient to provide at least 2.5% $P_2O_7^{4-}$, the toothpaste being characterised by having a pH of at least 7.6 which rises by at least 0.45 on dilution of the toothpaste with distilled carbon-dioxide free water in the proportion of 10g of toothpaste to 30ml of water.

2. A toothpaste as claimed in claim 1 which has a pH of at least 8.0.

3. A toothpaste as claimed in claim 1 or claim 2 wherein the pH of the toothpaste on dilution in the manner specified in claim 1 rises by at least 0.60.

4. A toothpaste as claimed in any of the preceding claims wherein the abrasive agent is silica.

5. A toothpaste as claimed in any of the preceding claims wherein the abrasive agent is present in an amount of 5 to 50% by weight.

6. A toothpaste as claimed in any of the preceding claims wherein the tetrapyrophosphate salt consists of a mixture of tetrapotassium pyrophosphate and tetrasodium pyrophosphate.

7. A toothpaste as claimed in claim 6 wherein the weight ratio of tetrapotassium pyrophosphate to tetrasodium pyrophosphate is 1.2:1 to 3.7:1.

8. A toothpaste as claimed in any of the preceding claims also comprising a fluoride-ion source sufficient to supply from 50ppm to 350ppm of fluoride ions.

9. A toothpaste as claimed in claim 8 wherein the fluoride-ion source is sodium fluoride.

10. A toothpaste as claimed in any of the preceding claims wherein the toothpaste is glycerol-free.

11. A method of preparing a composition as claimed in any one of claims 1 to 9 which comprises mixing together the components specified in the said claims.

**Revendications**

1. Pâte dentifrice servant à inhiber la formation des calculs dentaires, qui comprend un agent abrasif et un tetrapyrophosphate en une quantité suffisante pour introduire au moins 2,5% de $P_2O_7^{4-}$, la pâte dentifrice étant caractérisée en ce qu'elle présente un pH d'au moins 7,6 qui monte d'au moins 0,45 lors de la dilution de la pâte dentifrice avec l'eau distillée exempte d'anhydride carbonique en une proportion de 10 g de pâte dentifrice pour 30 ml d'eau.

2. Pâte dentifrice selon la revendication 1 dont le pH est d'au moins 8,0.

3. Pâte dentifrice selon la revendication 1 ou 2, dans laquelle le pH de la pâte dentifrice lors de la dilution sur le mode décrit dans la revendication 1, monte d'au moins 0,60.

4. Pâte dentifrice selon l'une quelconque des revendications précédentes, dans laquelle l'agent abrasif est une silice.

5. Pâte dentifrice selon l'une quelconque des revendications précédentes, dans laquelle l'agent abrasif est présent en une quantité de 5 à 50% en poids.

6. Pâte dentifrice selon l'une quelconque des revendications précédentes, dans laquelle le tetrapyrophosphate consiste en un mélange de tetrapyrophosphate de potassium et de tetrapyrophosphate de sodium.

7. Pâte dentifrice selon la revendication 6, dans laquelle le rapport pondéral du tetrapyrophosphate de potassium au tetrapyrophosphate de sodium est compris entre 1,2:1 et 3,7:1.

8. Pâte dentifrice selon l'une quelconque des revendications précédentes, qui comprend également une source d'ion fluorure en une quantité suffisante pour introduire de 50 à 350 ppm d'ion fluorure.

9. Pâte dentifrice selon la revendication 8, dans laquelle la source d'ion fluorure est le fluorure de sodium.

10. Pâte dentifrice selon l'une quelconque des revendications précédentes, dans laquelle la pâte dentifrice est exempte de glycérol.

11. Procédé de préparation d'une composition selon l'une quelconque des revendications 1 à 9, qui consiste à mélanger ensemble les composants indiqués dans lesdites revendications.

**Patentansprüche**

1. Zahnpasta zur Verhinderung der Bildung von Zahnstein, die ein Schleifmittel und eine Menge an Tetrapyrophosphatsalz umfaßt, die ausreicht um mindestens 2,5 % $P_2O_4^{4-}$ zu liefern, wobei die Zahnpasta dadurch gekennzeichnet ist, daß sie einen pH-Wert von mindestens 7,6 hat, der bei Verdünnung der Zahnpasta mit destilliertem kohlendioxidfreiem Wasser im Verhältnis von 10 g auf 30 ml Wasser um mindestens 0,45 ansteigt.

2. Zahnpasta nach Anspruch 1, worin der pH-Wert mindestens 0,0 beträgt.

3. Zahnpasta nach Anspruch 1 oder 2, worin der pH-Wert der Zahnpasta nach Verdünnung wie in Anspruch 1 angegeben, mindestens um 0,60 steigt.

4. Zahnpasta nach einem der vorhergehenden Ansprüche, worin das Schleifmittel Kieselerde ist.

5. Zahnpasta nach einem der vorhergehenden Ansprüche, worin das Schleifmittel in einer Menge von 5 bis 50 Gew.-% vorhanden ist.

6. Zahnpasta nach einem der vorhergehenden Ansprüche, worin das Tetrapyrophosphatsalz aus einem Gemisch aus Tetrakaliumpyrophosphat und Tetranatriumpyrophosphat besteht.

7. Zahnpasta nach Anspruch 6, worin das Gewichtsverhältnis von Tetrakaliumpyrophosphat zu Tetranatriumpyrophosphat 1,2:1 bis 3,7:1 beträgt.

8. Zahnpasta nach einem der vorhergehenden Ansprüche, die auch eine Fluoridionenquelle umfaßt, die ausreicht um 50ppm bis 3500ppm Fluoridionen zu liefern.

9. Zahnpasta nach Anspruch 8, worin die Fluoridionenquelle Natriumfluorid ist.

10. Zahnpasta nach einem der vorhergehenden Ansprüche, worin die Zahnpasta kein Glyzerin enthält.

11. Verfahren zur Herstellung einer Zusammensetzung nach einem der Ansprüche 1 bis 9, das das Vermischen der in den genannten Ansprüchen angegebenen Bestandteile umfaßt.